# EUROPEAN PATENT APPLICATION

(11) **EP 2 862 512 A1**
(43) Date of publication of application: **22.04.2015**
(21) Application number: 13807302.8
(22) Date of filing: 14.06.2013
(51) Int. Cl.: A61B 5/05, G06F 19/26

(54) **MULTIMEDIA TERMINAL WITH MEASUREMENT OF VITAL PARAMETERS**

(30) Priority: 18.06.2012 ES 201230951
(71) Applicant: MEDIP HEALTH, S.L., 46722 Beniarjó Valencia (ES)
(72) Inventor: CREMADES PERIS, Francisco Javier, E-46722 Valencia (ES); ESTEVAN SASTRE, Cándido, E-46722 Valencia (ES); MORO CALVO, Carlos, E-46722 Valencia (ES); VICENTE TOBAR, Daniel, E-46722 Valencia (ES); BARBER VIDAL, Constantino José, E-46722 Valencia (ES); CREMADES CREMADES, Jorge, E-46722 Valencia (ES); TRIBALDOS HERVÁS, Miguel Ángel, E-46722 Valencia (ES); ORTEGA GONZÁLEZ, Miguel, E-46722 Valencia (ES); ASTRUGA ABAD, David, E-46722 Valencia (ES)
(74) Representative: Schäfer, Matthias W.
(86) International application number: PCT/ES2013/070390
(87) International publication number: WO 2013/190157

(57) **Abstract**

Multimedia terminal with vital parameters monitoring, commonly used in hospitals and homes to enable access to communication networks, TV and multimedia contents to bed-ridden persons. It also comprises a multichannel measurement module showing readings on the same screen, including electronic circuits and connection for probes for electrocardiograms, pulse oximeters, tensiometers, temperature sensors, etc.

The main advantage of this invention is that it allows to measure, save and send vital parameters with the same multimedia device in each room, and to input values automatically and simultaneously into patients' medical histories in a cost-effective and comfortable way, without moving equipment between patients' rooms.

## Description

As indicated in the title, this description relates to a multimedia terminal with vital parameters monitoring. On one hand, this device will allow patients to have access to communication networks, TV, multimedia content and other entertainment services. On the other, it will allow medical practitioners to measure patients' vital signs, which are simultaneously recorded in their medical history. This terminal is aimed for use mainly in hospitals and homes.

This terminal simultaneously combines entertainment services through multimedia contents such as TV, radio, internet, communication networks, telephone, videoconference, films and music, among others, as well as a series of medical functions, such as vital signs monitoring or access to medical histories. It mainly consists of a support mounted onto the wall, ceiling, floor or bed, which includes all main electronic circuits, and an articulated arm with several sections, which allows medical practitioners and patients to have an easier and more personalized use, although the plan is to make the terminal autonomous.

The interconnection wiring is inside this articulated arm, which ends in a user terminal, with a preferably LCD, LED, TFT, etc. touchscreen and graphic display, an overlapping touch keyboard, loudspeakers, videocamera, microphone, one or many connectors for digital multimedia devices, warning lights and telephone. Electronic circuit systems with wireless technology and specific connectors for electrocardiograms, pulse oximeters, tensiometers, temperature sensors, etc. are fitted in the screen. These are able to measure vital signs through multiple channels and show patients' real-time information on the screen.

It also has screen user identification with a magnetic card, barcode reader, radio frequency identification (RFID) chip, or manually introducing a personal code.

### Background of the Invention

Currently, there are many widely known devices that provide multimedia content for bed-ridden persons. The simplest devices, like the one described in Utility Model 9901212 'Screen support device,' only place a TV set showing multimedia content on the wall in front of the patient. These devices are fixed and do not allow a personalized use, nor give access to authorized personnel alone, showing confidential and restricted information to the patient or any other person in the room, even more so considering that most hospital rooms are shared.

There are more complex devices, like Chinese Patent No. 101408995 'Hospital examination-distribution queuing system with multimedia play function,' which is composed of a number of display screens combining an examination-distribution queuing system for public hospitals with a multimedia playing system that patients can watch while waiting.

There have been more advanced developments combining several multimedia features for hospital and home patients, like Spanish Patent No. 9801482 'Service control and management system in building rooms' or Japanese Patent No. 2003242252 'Sickroom support system, sickroom television and sickroom support program.' Besides other devices commercially available including an articulated support for television sets near the patient. These devices have issues mainly in the articulated arms, which require sophisticated reinforcements on walls or ceilings and expensive and complicated articulation and support mechanisms due to the heavy weight and constant movement of the monitor, screen, loudspeakers and electronics together. These reinforcements and mechanisms turn them into rigid and hardly operating devices prone to breakdowns and holdups both for mounting on a wall of fitting to a bed. Besides, adding the monitor or even a computer at the end of the arm creates a source of heat and noise near patients, which is usually negative for their recovery.

There have been some attempts to make devices lighter, such as European Patent No. 89123084 'Control apparatus.' In it, however, controls are included in one unit at the end of the articulated arm, not combining all multimedia features.

Utility Model U201030643 'Articulated multimedia terminal' offers further developments addressing most of the previous issues but, like all previous devices, it does not include vital signs monitoring, making it necessary to resort to external equipment.

There are many devices available for vital signs monitoring, and many of them are portable. Some examples include Patent ES8500037 'Portable E.C.G. recording apparatus,' Patent ES21855488 'Cardiovascular disease detection and monitoring via mobile technology' and Utility Model ES1047988 U 'Portable electrocardiograph based on an expander electronic module for handhelds, able to record, process, represent and transmit heart rate.' However, these are all conventional independent devices, with no multimedia terminal.

There are many other conventional independent devices with no multimedia terminal. Some more examples include Utility Model ES0210350 U 'Portable blood pressure meter,' Patent ES2342476 'Sphygmomanometer,' Patent ES0285887 'Electronic sphygmomanometer to automatically measure systolic and diastolic blood pressure, and differences in light and acoustic readings, for medical purposes,' Patent ES2342945 / WO2005087094 'Pulse oximeter with separate ensemble averaging for oxygen saturation and heart rate' and Utility Model ES1076877 U 'Monitoring device for newborns.'

Lastly, there are some well-known devices to measure vital signs such as the ones described in Patent ES2029410 ' Improvements in public devices for body measurements,' and Patent ES0429001'Device for constant monitoring of a patient's vital parameters.' Neither device includes multimedia features.

It is worth noticing that there is long-standing technical preconception, still existing today, about the fact that vital sign monitoring devices, and medical equipment in general, should be specialized and free of any non medicine- or life support-related features, such as multimedia contents for entertainment purposes. Therefore, there currently are several monitoring devices for outpatients and hospital patients, and several devices with multimedia entertainment contents, but as completely separate equipment, with no connection between them.

### Description of the Invention

This multimedia terminal with vital parameters monitoring has been devised in order to solve the existing issue of combining vital signs measuring with multimedia entertainment contents for bed-ridden persons at hospitals and homes, as well as to integrate new technologies in hospitals and homes while reducing the cost of equipment. The terminal simultaneously combines entertainment services through multimedia contents such as TV, radio, internet, communications networks, telephone, videoconference, films, and music, among others, as well as a series of medical functions such as vital signs monitoring or access to medical histories. It mainly consists of a support mechanically mounted onto the wall, ceiling, floor or bed, which fixes the equipment and includes all main electronic circuits, and an articulated arm with several sections and interconnection wiring inside, ending in a user terminal.

There are plans for the user terminal to be autonomous and independent from the circuits on the wall through a wireless connection, depending on set and desired technical settings.

In turn, this user terminal comprises a flat screen with graphic display and an overlapping touch keyboard, preferably a LCD, LED, TFT, etc. touchscreen, loudspeakers, videocamera, microphone, telephone and one or many connectors for digital devices, preferably USB, FLASH memory cards or both, as well as user identification means.

Should the terminal become autonomous, the necessary circuits will be fitted so that it does not depend on the module on the wall, ceiling or floor for electric supply and IT connection.

The terminal also comprises a multichannel measurement module showing readings together with the patient's medical history, since its electronic circuits and communication media are connected to probes for full electrocardiograms, pulse oximeters, tensiometers, temperature sensors, etc. Alarms or alerts may be programmed for various measurements. It will also allow wireless connection through Bluetooth to other external medical devices (electrocardiographs, x-rays, ultrasound scanners) simultaneously recording the data provided by said external devices into patients' medical history.

The terminal is a multifunctional device for home, business and mainly hospital use, combining infrastructure, technology and services as well as reducing costs in wiring, technologies, devices and procedures. It is used with two purposes. In the hospital room, the device is an entertainment multimedia platform with access to radio and TV channels, telephone for internal and external calls, internet and email, games, ebook reader, etc. As a support equipment for healthcare staff, it is an IT station with access to all hospital applications (without having to resort to additional devices), and hence to patients' real-time data from the bed (history, medication, prescription, etc.) as well as audio/video monitoring and surveillance of bed-ridden patients. The device includes a monitor with vital information, which measures patients' vital signs through probes and countless wireless probes. Data is displayed on the screen, managed and recorded in real-time on patients' medical history without any additional devices, saving time and money as well as avoiding medical errors when transcribing them.

Besides, the terminal only allows access to medical functions to dully authorized personnel.

The device smartly identifies which vital sign probe has been connected. It has smart and identical ports to connect any compatible probe, without having to use one specific port for one specific probe.

The device also has a repository of vital signs that automatically detects the units and probes that have been connected, and automatically records and saves all the data received from them. The repository and the device monitor, trace and follow-up vital signs and patients' evolution, including minimum and maximum values for each vital sign, as well as an alarm triggered when these values are exceeded.

The device allows to remotely monitor a patient through an internet connection even outside the intranet where it is set. Hence, any dully authorized third party may check vital signs in real-team for diagnosis and treatment.

In this way, a single device is used with two purposes. On one hand, as a multimedia device for patients' personal use with access to TV, radio, internet, communication networks, telephone, videoconference, films, music, etc. On the other, as a device for medical practitioners to have access to patients' vital signs (electrocardiograph, pulse oximeter, tensiometer, etc.). So, for instance, there will be an electrocardiograph next to each patient permanently connected to their medical history, which is actually where it is most needed.

This device is noninvasive in monitoring vital signs, so it complements but does not replace life support equipment.

### Advantages of the Invention

This multimedia terminal with vital signs monitoring has several advantages over the devices currently available. The most important one is that it simultaneously combines multimedia content for entertainment purposes, such as TV, radio, internet, communications networks, telephone, videoconference, films and music, among others, with a series of medical functions, such as vital signs monitoring or access to medical histories.

It is worth noticing that there is a long-standing technical preconception, still existing today, about the fact that vital sign monitoring devices, and medical equipment in general, should be specialized and free from any non medicine- or life support-related features, such as multimedia contents for entertainment purposes.

Another advantage is the considerable improvement achieved by using the product compared to multimedia solutions for entertainment purposes alone and to conventional medical solutions, since there is a larger return on investment - a device for vital signs monitoring at the bedside, instead of the one that is moved from one bed to another, at a fraction of the cost.

Since the multimedia device in each room is connected to the hospital's IT network, gives access to the medical history of the patient being treated and measures vital signs, the data provided to patients is more reliable and done in a more secure, economic and comfortable way, as devices are not moved between rooms and avoiding human errors when manually inputting data in patients' digital medical histories.

Another important advantage is that its cost is reduced by more than 80% because electronic circuits, supports, screen and envelopes are shared with the multimedia equipment, allowing to have one device per patient.

The advantage for medical staff, who do not need to move devices from one room to the other, is noteworthy. This reduces the number of hours of work, breakdowns, dead batteries, delays, reliable data input, etc., contributing to an improvement in the quality of assistance and a significant reduction in the costs of hospitalization.

Another important advantage is displaying patients' medical histories on the screen allowing medical staff to input and update data in real-time. This in turn improves treatments and measurements, making them more reliable and avoiding possible human errors when manually noting data down in order to later manually input them in patients' medical histories.

Another essential advantage is that the multimedia terminal provides a maximum level of security when processing medical data. When patients are admitted into hospital, they are allocated a room and file number, with a numeric code and, in many cases, an encrypted, untransferable and secure code, like a barcode. This multimedia terminal is always in the same room where the device is located, thus avoiding any possible human error when inputting patients' data. There is no way to access another patient's data from this terminal. Only authorized personnel with an encrypted code and following the hospital's internal protocols can access to modify data.

Another advantage compared to mobile devices currently used in hospitals is that this terminal is fixed and permanently connected to the hospital's IT and electric networks, allowing to access and process medical history data at maximum speed and avoiding unexpected power cuts or lack of wireless coverage .

It is worth highlighting the advantage for medical staff when accessing a patient's medical history from the terminal, since it is shown directly on the screen without having to look for it, making it 100% reliable and much quicker than any other existing device. This means that medical treatments will be more efficient, the time attending patients will be reduced and costs will be significantly cut.

There is another great advantage for medical staff: all connectors are alike and compatible regardless of use, so probes can be connected to any of them, since the terminal will identify the type of probe, activate its application and show it on screen avoiding any possible errors as with current devices.

It is also important to note that alarms or alerts may be programmed for various measurements.

Another very important advantage is that, by including vital signs or parameters readings in the multimedia user terminal, data collected is saved in medical histories and later analyzed by professionals. In this way, doctors have access to huge amounts of real-time information to process and treat accordingly, which cannot be done with the existing conventional equipment. Additionally, this data can be sent to database control and treatment centers to centralize and process it without affecting patients' comfort in hospital, since vital signs measuring can be perfectly combined with using the screen for entertainment purposes.

Another advantage is that the design of the device easily and economically allows patients to access TV, radio, internet, communication networks, telephone, videoconference, films and music, among others, without leaving the bed or using a remote control. The touchscreen totally controls both vital signs monitoring and multimedia services, although an additional remote control may be introduced if necessary.

Another advantage of this invention is that it consists of a light support, easy to handle by any sick or bed-ridden person, without using much strength or even help from others, and adaptable to any position in case of patients with disabilities or mobility difficulties.

Lastly, the user terminal is flat, even super-flat, so it is really nonintrusive and does not give an overwhelming or complex feeling like most conventional devices. Also, since it is all in a multimedia environment, measurement devices are friendlier, creating a more entertaining and pleasant atmosphere, particularly for children.

Finally, outside hospitals, it may be used for home hospitalization with medical remote control, as long as the terminal is connected to a network at the sick person's home.

### Description of Figures

In order to better understand the current invention, a preferred practical embodiment of a multimedia terminal with vital signs monitoring is provided in the enclosed drawings.

Among the drawings, figure -1- shows the whole device, with the measurement module in the user terminal.
Figure -2- shows a detail of the back side of the user terminal with the measurement module fitted by means of an additional envelope, being the measurement module in the user terminal in the version with cable connectors.
Figure -3- shows a detail of the back side of the user terminal with the measurement module fitted into it, being the measurement module in the user terminal in the version with cable connectors.
Figure -4- shows the whole device, with the measurement module in the terminal's support.
Figure -5- shows an example of how the measurement module and electrocardiogram, blood pressure and pulse oximeter probes can be connected to the patient, being the measurement module in the user terminal in the version with cable connectors.
Figure -6- shows an example of how only measurements are displayed on the user terminal.
Figure -7- shows an example of how measurements are displayed together with multimedia programming on the user terminal.
Figure -8- shows an example of how measurements are displayed on top of multimedia programming on the user terminal.
Figure -9- shows a simplified block diagram of the invention with the measurement module in the user terminal in the version with cable connectors.
Figure -10- shows a simplified block diagram of the invention with the measurement module in the user terminal in the version with wireless connection.
Figure -11- shows a simplified block diagram of the invention with the measurement module in the support in the version with cable connectors.
Figure -12- shows a simplified block diagram of the invention with the measurement module in the support in the version with wireless connection.

### Preferred Embodiment of the Invention

This terminal simultaneously combines entertainment services through multimedia contents such as TV, radio, internet, communication networks, telephone, videoconference, films and music, among others, as well as a series of medical functions, such as vital signs monitoring or access to medical histories. As shown in the enclosed drawings, it mainly consists of a user terminal (5), preferably linked to a support (1) mounted onto a wall or bed, which includes electronic circuits and a horizontal articulated arm made up of at least two sections (2) with pivot points (3) linking them and with the support (1), and interconnection wiring (4) inside. There are plans for the user terminal to be autonomous and independent from the circuits on the wall through a wireless connection, depending on set and desired technical settings.

This user terminal (5) also comprises a flat screen (6) with graphic display and an overlapping touch keyboard, loudspeakers (7), videocamera (8), microphone (9), one or many connectors (10) for digital devices and user identification means (11,12,13). The electronic circuits of the support (1) comprise a motherboard (24) with a microprocessor, RAM and nonvolatile memory, a digital communications module (25) and a power supply (26).

The terminal consists of a multichannel measurement module (14) connected to the electronic circuits of the support (1) by means of interconnection wiring (4) showing measurement readings on the same flat screen (6) used for multimedia contents, including electronic circuits and connection for probes to run full ECG (18) and for auxiliary probes for vital signs monitoring (19).

The multichannel measurement module (14) may be placed on the back side of the user terminal (5) by means of an additional envelope (17) mounted on the user terminal (5), or fitted into the user terminal (5). Alternatively, the multichannel measurement module (14) may be placed on the support (1).

Probes to run electrocardiograms (18) can be connected by means of multipolar electrical connectors (15), wireless modules (28), or a combination of both. Likewise, auxiliary probes to measure vital parameters (19) can be connected by means of multipolar electrical connectors (16), wireless modules (29, 30), or a combination of both.

When connected, auxiliary probes to measure vital parameters (19) are automatically detected through an appropriate protocol. Auxiliary probes to measure vital parameters (19) may be pulse oximeters, tensiometers and temperatures sensors, although this may be extended to any other noninvasive probes.

The digital communications module (25) of the support (1) is connected to the hospital's server (27) to give access to patients' medical histories, allowing to automatically update the measurements taken.

User identification means may be a card reader (11), a fingerprint reader (12), a code reader (13), a radio frequency identification (RFID) chip, manually introducing a personal code on the touchscreen keyboard (22), or the combination of any of them.

This multimedia terminal with vital parameters monitoring has a characteristic operating procedure: a patient mode, in which the measurement module (14) is disabled and only the multimedia functions are enabled; and a doctor mode, where the measurement module (14) is enabled, and the readings are shown on the flat screen (6) and saved or sent to a database.

The device goes into doctor mode when medical staff identify themselves through user identification means (11, 12, 13). Once on doctor mode, when the auxiliary probes to measure vital parameters (19) are connected, the motherboard (24) connects with them through the measurement module (14) and automatically identifies them, carrying out the appropriate monitoring protocol.

On doctor mode, available in real-time and in different areas of the screen, are: readings (20) from probes to run electrocardiograms (18), readings (21) from auxiliary probes to measure vital parameters (19), the patient's medical history (23) obtained from the server (27), and a touchscreen keyboard (22) for data control, input or correction. This allows medical staff to see the values obtained, or to save or send data for later process or analysis. In this case the terminal is used as a data monitor, for instance, in the case of patients who cannot watch TV or are very sick. In this case, the user terminal may be against the wall and operate as a permanent data monitor alone.

Alternatively, on doctor mode and available in real-time and in different areas of the screen, are: readings (20) from probes to run electrocardiograms (18), readings (21) from auxiliary probes to measure vital parameters (19), the patient's medical history (23) obtained from the server (27), and a touchscreen keyboard (22) for data control, input or correction, together with multimedia programming (24), either separately or as a background to the readings, which would be overlapping it. In this way, patients' parameters can be monitored over a long period of time while they simultaneously have access to entertainment multimedia programs or functions, which is particularly interesting in the case of children or long-term monitoring.

Alternatively, while multimedia content is displayed, for instance a TV program, measurements can be made and saved without patients knowing, and readings will only appear on the screen if the nurse requests them or the doctor wants to see them. This allows patients to comfortably watch TV while data is being saved and sent without bothering them on the screen.

Alarms or alerts, both local and remote, may be programmed for various measurements.

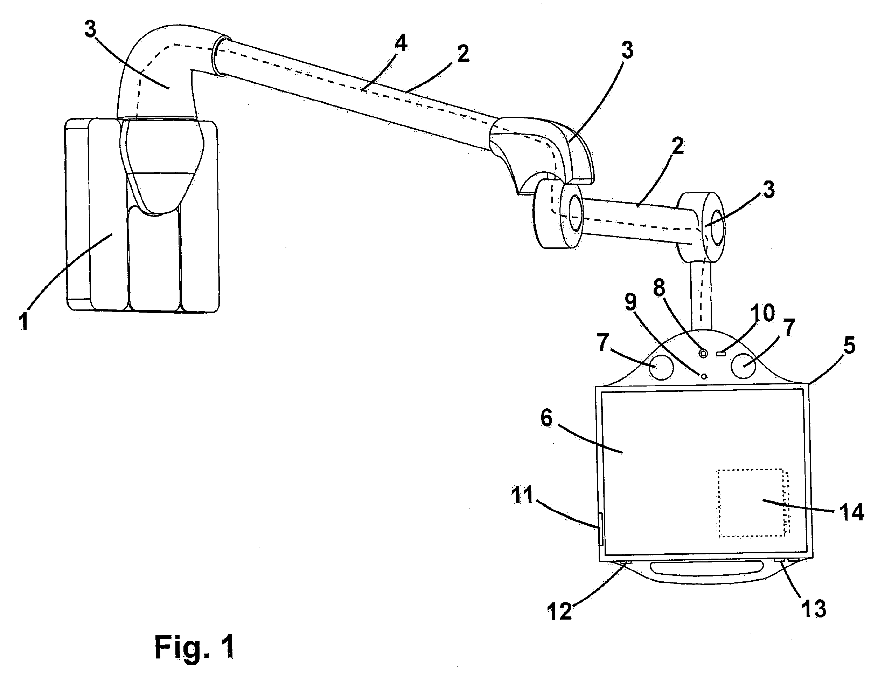

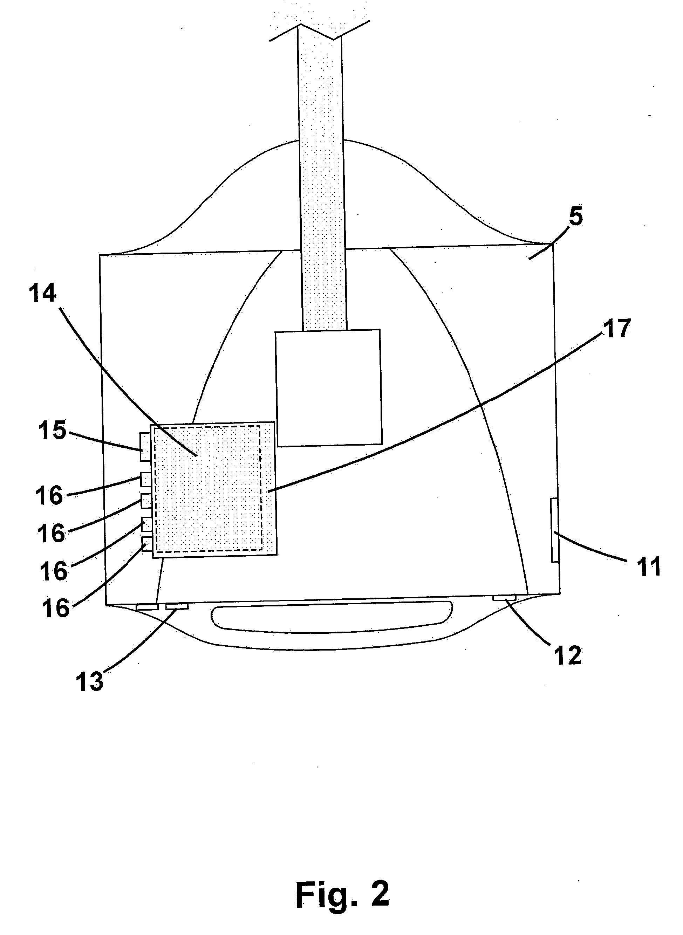

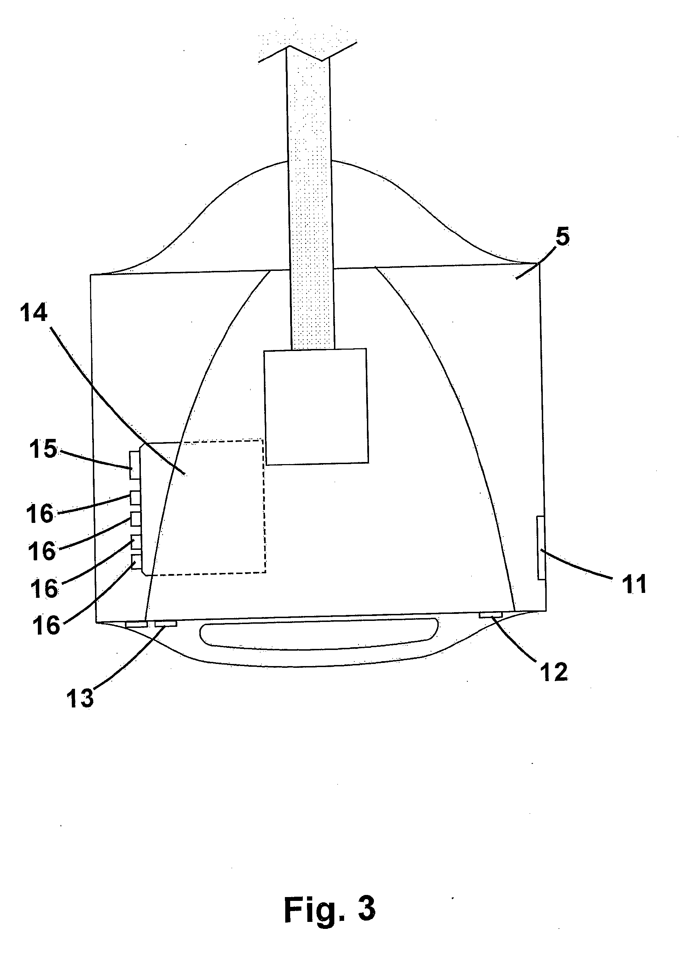

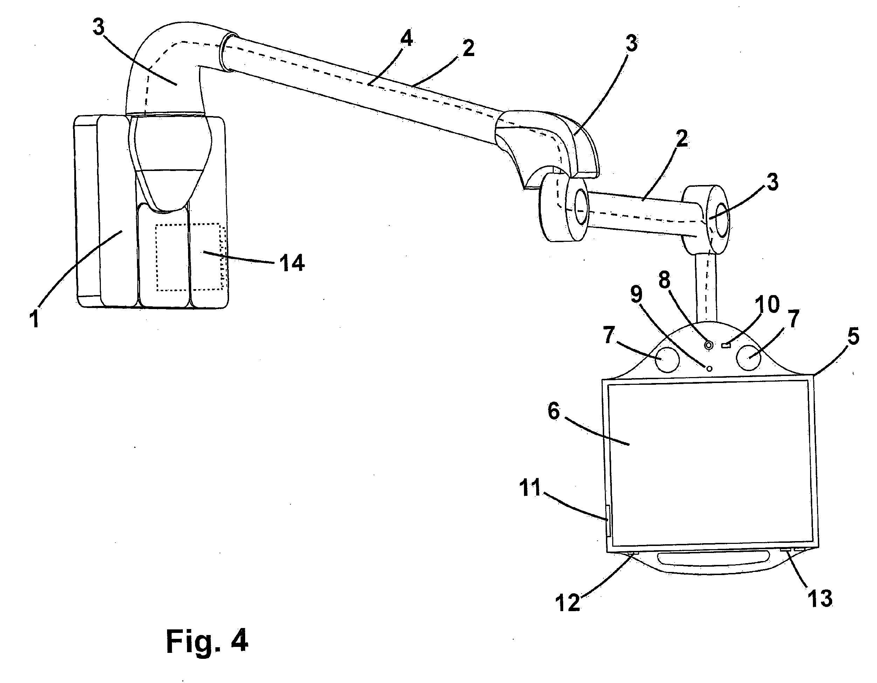

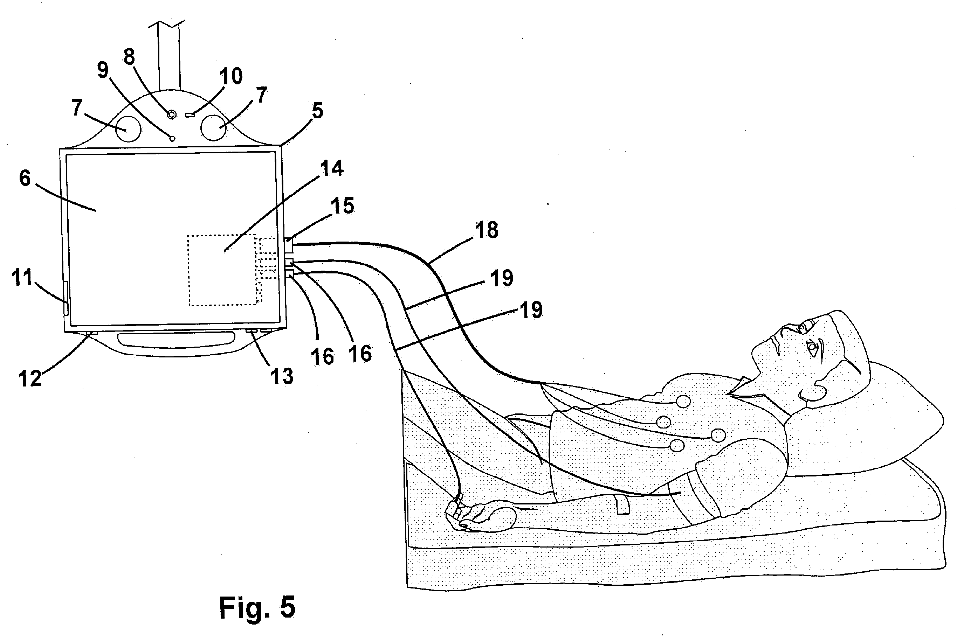

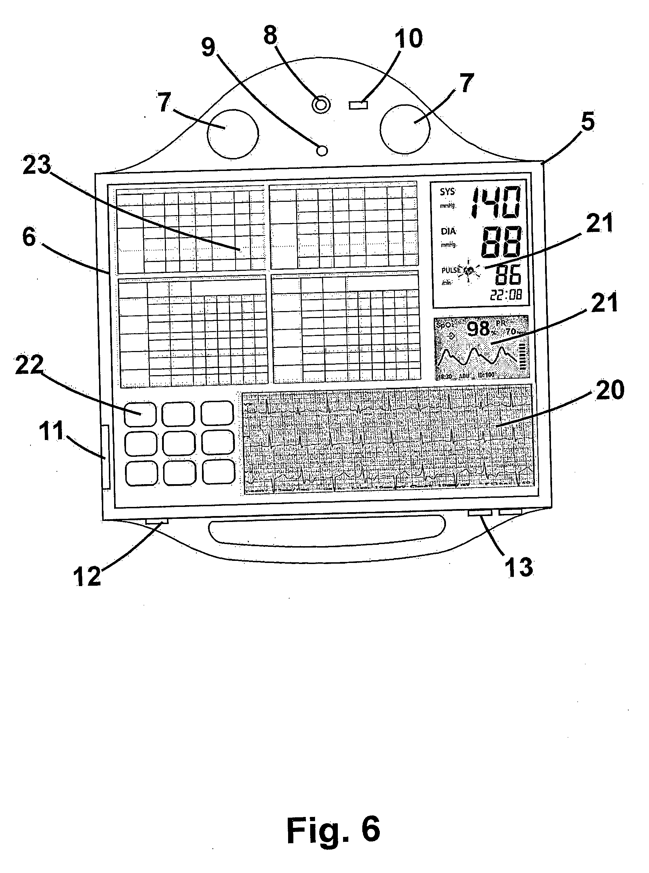

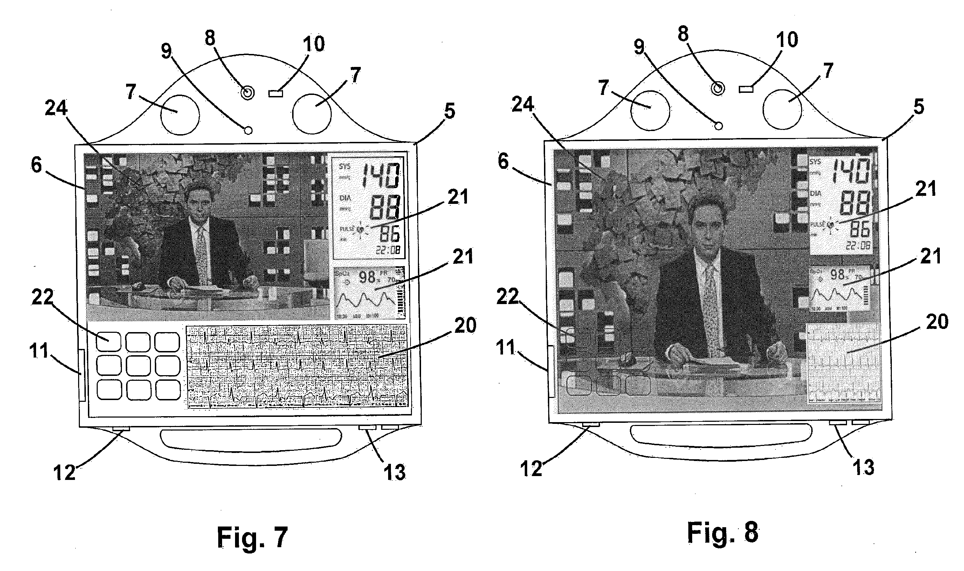

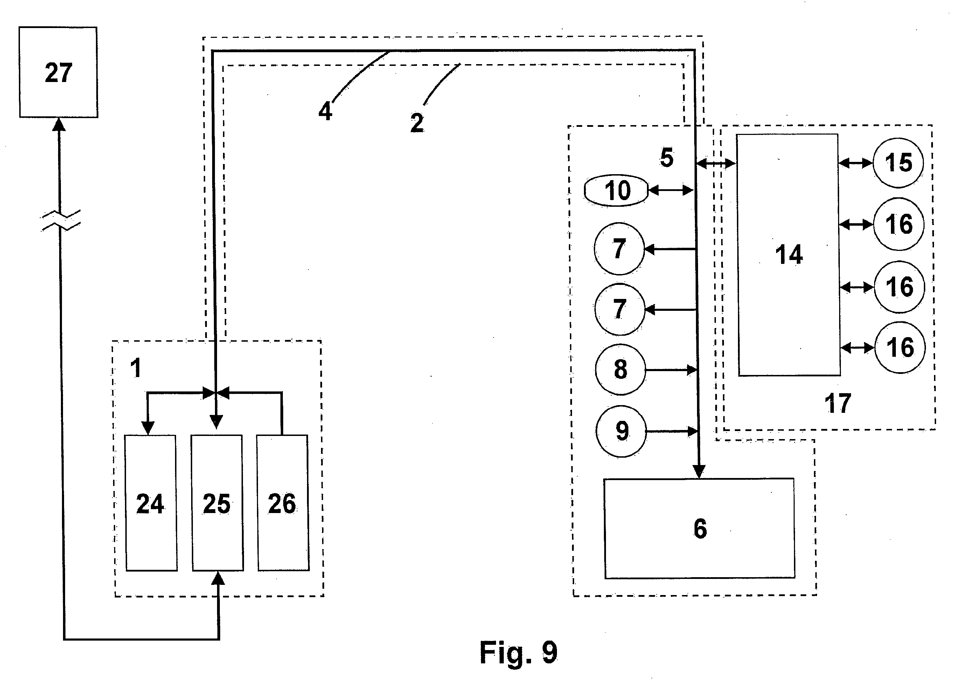

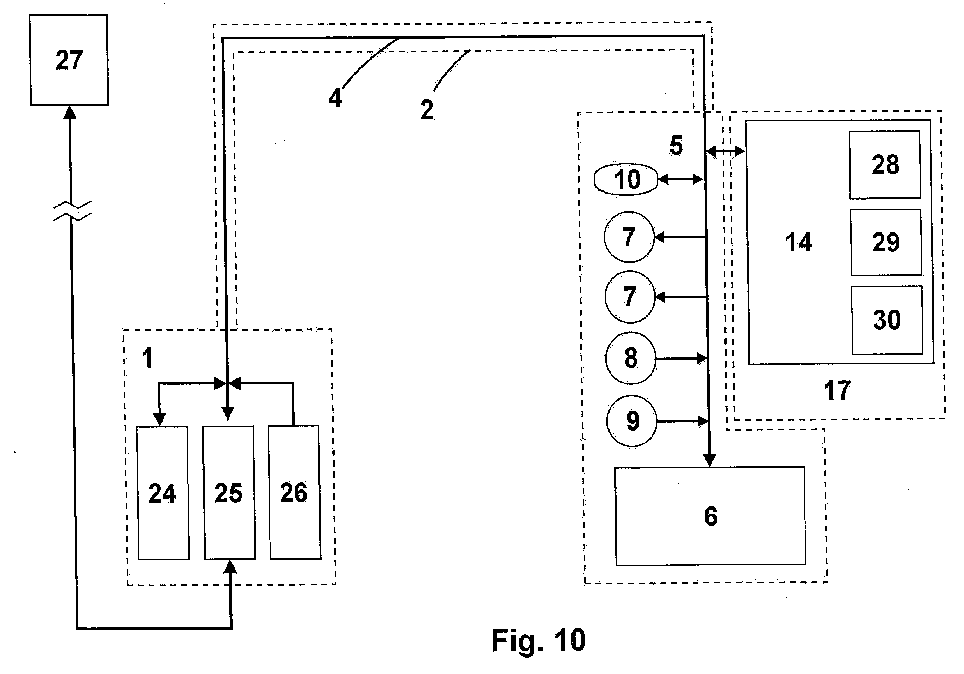

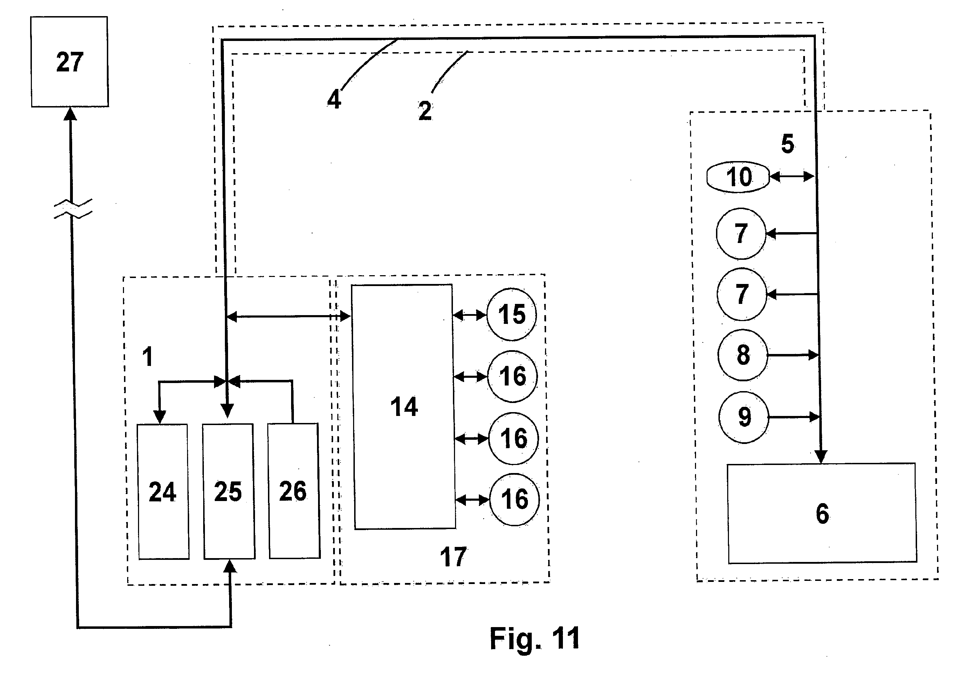

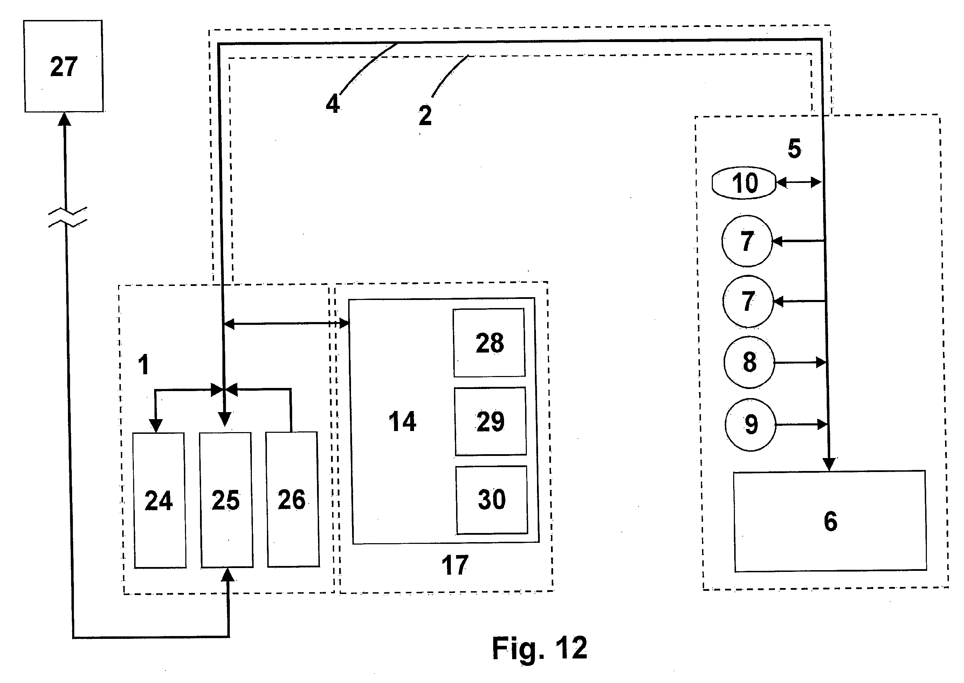

## Claims

1. Multimedia terminal with vital parameters monitoring, as commonly used in hospitals and homes to facilitate the access to communication networks and multimedia contents, such as TV, radio, internet, communication networks, telephone, videoconference, films and music, among others, for bed-ridden persons. Comprising a user terminal (5), which may be autonomous or linked to a support (1) mounted onto a wall or bed, which includes electronic circuits, and a horizontal articulated arm made up of at least two sections (2) with pivot points (3) linking them and with the support (1), and interconnection wiring (4) inside. This user terminal (5) also consists of a flat screen (6) with graphic display and an overlapping touch keyboard, loudspeakers (7), a videocamera (8), a microphone (9), one or many connectors (10) for digital devices and user identification means (11,12,13). The user terminal (5) or the support (1) may have a motherboard (24) with a microprocessor, RAM and nonvolatile memory, a digital communications module (25) and a power supply (26) **characterized by** a multichannel measurement module (14) connected to the electronic circuits of the user terminal (5) showing measurement readings on the same flat screen (6) tailored for multimedia contents, including electronic circuits and connection for probes to run full ECG (18) and to auxiliary probes for vital signs monitoring (19).

2. Multimedia terminal with vital parameters monitoring, according to claim 1, **characterized by** a multichannel measurement module (14) placed on the back side of the user terminal (5).

3. Multimedia terminal with vital parameters monitoring, according to claim 2, **characterized by** a multichannel measurement module (14) placed on the back side of the user terminal (5) by means of an additional envelope (17) attached to the user terminal (5).

4. Multimedia terminal with vital parameters monitoring, according to claim 1, **characterized by** a multichannel measurement module (14) placed on the support (1).

5. Multimedia terminal with vital parameters monitoring, according to claim 1, **characterized by** connection for probes to run electrocardiograms (18) by means of multipolar electrical connectors (15), wireless modules (28), or a combination of both.

6. Multimedia terminal with vital parameter monitoring, according to claim 1, **characterized by** connection for auxiliary probes to measure vital parameters (19) by means of multipolar electrical connectors (16), wireless modules (29,30), or a combination of both.

7. Multimedia terminal with vital parameters monitoring, according to claim 1, **characterized by** auxiliary probes to measure vital parameters (19) chosen from amongst pulse oximeters, tensiometers and temperature sensors.

8. Multimedia terminal with vital parameters monitoring, according to claim 1, **characterized by** the digital communications module (25) of the support (1) being connected to the hospital's server (27) to give access to patients' medical histories, allowing to automatically update the measurements taken.

9. Multimedia terminal with vital parameters monitoring, according to claim 1, **characterized by** user identification means being a card reader (11), a fingerprint reader (12), a code reader (13), a radio frequency identification (RFID) chip, manually introducing a personal code on the touchscreen keyboard (22), or similar.

10. Operating procedure of a multimedia terminal with vital parameters monitoring, as described in previous claims, **characterized by** a patient mode, in which the measurement module (14) is disabled and only the multimedia functions are enabled, and a doctor mode, where the measurement module (14) is enabled, and the readings are shown on the flat screen (6) and saved or sent to a database.

11. Operating procedure of a multimedia terminal with vital parameters monitoring, according to claim 10, **characterized by** the device being on doctor mode when medical staff identify themselves through user identification means (11, 12, 13).

12. Operating procedure of a multimedia terminal with vital parameters monitoring, according to claim 11, **characterized by** the fact that on doctor mode, when the auxiliary probes to measure vital parameters (19) are connected, the motherboard (24) connects with them through the measurement module (14) and automatically identifies them, carrying out the appropriate monitoring protocol.

13. Operating procedure of a multimedia terminal with vital parameters monitoring, according to claim 10, **characterized by** the fact that on doctor mode, available in real-time and in different areas of the screen, are: readings (20) from probes to run electrocardiograms (18), readings (21) from auxiliary probes to measure vital parameters (19), the patient's medical history (23), obtained from the server (27), and a touchscreen keyboard (22) for data control, input or correction.

14. Operating procedure of a multimedia terminal with vital parameters monitoring, according to claim 10, **characterized by** the fact that on doctor mode, available in real-time and in different areas of the screen are: readings (20) from probes to run electrocardiograms (18), readings (21) from auxiliary probes to measure vital parameters (19), the patient's medical history (23), obtained from the server (27), and a touchscreen keyboard (22) for data control, input or correction, together with multimedia programming (24), either separately or as a background to the readings, which would be overlapping it.

15. Operating procedure of a multimedia terminal with vital parameters monitoring, according to claim 10, **characterized by** the fact that on doctor mode only multimedia contents (24) are available, readings (20) from probes to run electrocardiograms (18), readings (21) from auxiliary probes to measure vital parameters (19), and the touchscreen keyboard (22) for data control, input or correction are hidden and only show when medical staff identify themselves.
